# EUROPEAN PATENT APPLICATION

(11) **EP 1 568 380 A1**
(43) Date of publication of application: **31.08.2005**
(21) Application number: 03777222.5
(22) Date of filing: 04.12.2003
(51) Int. Cl.: A61K 45/00, A61K 31/7056, A61K 31/706, A61P 3/04, A61P 43/00

(54) **PREVENTIVE OR REMEDY FOR DISEASES CAUSED BY HYPERGLYCEMIA**

(30) Priority: 04.12.2002 JP 2002352201
(71) Applicant: Kissei Pharmaceutical Co., Ltd., Nagano-Pref. 399-8710 (JP)
(72) Inventor: ITO, F., Kissei Pharmaceutical Co., Ltd., Central, Minamiazumi-gun, Nagano 399-8304 (JP); SHIBAZAKI, T., Kissei Pharm. Co., Ltd., Central, Minamiazumi-gun, Nagano 399-8304 (JP); TOMAE, M., Kissei Pharmaceutical Co., Ltd, Central, Minamiazumi-gun, Nagano 399-8304 (JP); FUSHIMI, N, Kissei Pharmaceutical Co, Ltd, Central, Minamiazumi-gun, Nagano 399-8304 (JP); ISAJI, M., Kissei Pharmaceutical Co., Ltd, Central, Minamiazumi-gun, Nagano 399-8304 (JP)
(74) Representative: Hayes, Adrian Chetwynd
(86) International application number: PCT/JP2003/015503
(87) International publication number: WO 2004/050122

(57) **Abstract**

The present invention provides pharmaceutical compositions comprising as an active ingredient a selective SGLT1 inhibitor (e.g., an SGLT1 inhibitor substantially showing no GLUT2 and/or GLUT5 inhibitory effect), which have a wider range of inhibitory effect on carbohydrate absorption and a hypoglycemic effect caused by fructose ingestion at regular diets and therefore can exhibit a marked hypoglycemic effect, and which are suitable as agents for the prevention or treatment of diseases associated with hyperglycemia (e.g., diabetes, impaired glucose tolerance, diabetic complications, obesity).

## Description

### Technical Field

The present invention relates to an agent for the prevention or treatment of a disease associated with hyperglycemia, comprising as an active ingredient a selective sodium-dependent glucose transporter (hereinafter referred to as SGLT) 1 inhibitor.

More particularly, the present invention relates to an agent for the prevention or treatment of a disease associated with hyperglycemia, comprising as an active ingredient an SGLT1 inhibitor substantially showing no inhibitory effect on absorbing fructose through the small intestine, for example, an SGLT1 inhibitor substantially showing no facilitative glucose transporter (hereinafter referred as to GLUT) 2 and/or GLUT5 inhibitory effect.

### Background Art

In recent years, development and application of various agents for the treatment of diabetes have been progressing with the background of rapid increase of patients with diabetes and of necessity of strict blood sugar level control confirmed by large-scale clinical trials (e.g., see the following References 1-3). For example, α-glucosidase inhibitors such as acarbose, miglitol and voglibose, which delay carbohydrate absorption by prevention of carbohydrate digestion at the small intestine, are used to improve postprandial hyperglycemia. It has been reported that blood glucose level and HbA1c were significantly improved with application of these agents to patients with type 2 diabetes (e.g., see the following References 4-6). It has been also reported that acarbose, one of α-glucosidase inhibitors , has an effect on preventing or delaying the incidence of diabetes by applying to patients with impaired glucose tolerance (e.g., see the following Reference 7). Direct ingestion of monosaccharide such as glucose is increased with recent change of meal carbohydrate composition. However, α-glucosidase inhibitors do not inhibit monosaccharide absorption (e.g., see the following Reference 8). Therefore, it has been desired to develop agents which exert a wider range of inhibitory effect on carbohydrate absorption.

It has been known that SGLT1 exists in the small intestine, which controls carbohydrate absorption. It has been also reported that glucose and galactose malabsorption arises in patients with dysfunction due to congenital abnormalities of human SGLT1 (e.g., see the following References 9-11). In addition, it has been confirmed that SGLT1 is involved in glucose and galactose absorption (e.g., see the following References 12 and 13). Furthermore, carbohydrate digestion and absorption are generally increased in diabetes. For example, in OLETF rats and rats with streptozotocin-induced diabetic symptoms, it has been confirmed that expression of SGLT1 mRNA and protein levels are increased, and absorption of glucose and the like is accelerated (e.g., see the following References 14 and 15). It has been also confirmed that mRNA and protein of SGLT1 are highly increased in the human small intestine (e.g., see the following Reference 16). Therefore, SGLT1 inhibitors inhibit absorption of carbohydrates such as glucose at the small intestine, subsequently can prevent elevation of blood glucose level. Especially, it is considered that SGLT1 inhibitors are effective in normalizing postprandial hyperglycemia based on blocking or delaying carbohydrate absorption by the mechanism mentioned above.

It has been well known phlorizin is an SGLT inhibitor. In addition, Phlorizin has been known to decrease blood glucose level by enhancement of urinary glucose excretion (e.g., see the following References 17-20). However, phlorizin orally administered does not have this effect, because of degradation by β-glucosidase that exists in the gastrointestinal tract (e.g., see the following References 21-22).

It has been known that fructose at excess dosage beyond physiological condition leads to abnormal metabolism of lipid, purine and copper (e.g., see the following Reference 23). In recent years, it has been reported that small amounts of dietary fructose prevents elevation of blood glucose level in human, dogs and rats (e.g., see the following References 24-28). It is considered that these effects are based on enhancement of glucose uptake and of glycogen accumulation, and suppression of glucose production in liver (e.g., see the following References 24, 25, 27 and 29). In detail, fructose absorbed through the small intestine is taken in hepatocytes and converted to fructose-1-phosphate by fructokinase, and is translocated to nucleus. Glucokinase, which is known to decrease in patients with diabetes, exists in nucleus as inactive form complexed with the regulatory protein and fructose-6-phopsphate. The flucotose-6-phosphate in glucokinase complex is displaced by fructose-1-phosphate. Accompany to this displacement, the glucokinase is released from the regulatory protein to become active and then translocated to the cytosol. Activated glucokinase converts glucose to glucose-6-phosphate. Thus, acceleratedutilization of glucose results in increase of hepatic glucose uptake (e.g., see the following Reference 23). In addition, it has been reported that simultaneous administration of fructose reduced plasma insulin level in glucose tolerance tests using human and dogs (e.g. , see the following References 24 and 26). As mentioned above, small amounts of fructose have the effect of enhancing liver glucose uptake and glycogen accumulation, and of reducing insulin level. Therefore, it is considered that it can exert various effects such as improvement of glycogen synthesis which is lowered in patients with diabetes, lowering risk of macroangiopathy and protection of exhausted pancreatic beta cells due to postprandial hyperglycemia in addition to lowering blood glucose level in patients with diabetes.

An object to be solved in the present invention is to develop novel agents having a wider range of inhibitory effect on carbohydrate absorption at the small intestine, which are suitable for use in the prevention or treatment of a disease associated with hyperglycemia.

As mentioned above, phlorizin is an agent having an inhibitory effect on SGLT, and is degraded rapidly to phloretin by β-glucosidase that exists in the gastrointestinal tract (e.g., see the following References 30 and 31). It has been known that phloretin inhibits GLUT (e.g., see the following Reference 32). Thus, in the gastrointestinal tract, phlorizin has an inhibitory effect not only on SGLT but also on GLUT. It has been also known that, in small intestinal epithelial cells, GLUT5 is localized at the brush border membrane in small intestinal luminal side, and GLUT2 is localized at the basolateral membrane in capillary vessel side, and these GLUTs are involved in fructose absorption at the small intestine (e.g., see the following Reference 33).

The present invention is to provide a novel agent for the prevention or treatment comprising as an active ingredient a selective SGLT1 inhibitor, which is effective for a disease associated with hyperglycemia and can exert an effect caused by fructose ingestion.
Reference 1: The Diabetes Control and Complications Trial Research Group, N. Engl. J. Med., 1993.9, Vol.329, No.14, pp.977-986;
Reference 2: UK Prospective Diabetes Study Group, Lancet, 1998.9, Vol.352, No.9131, pp.837-853;
Reference 3: Makoto, TOMINAGA, Endocrinology & Diabetology, 2001.11, Vol.13, No.5, pp.534-542;
Reference 4: Hiroshi, MIYASHITA and 8 persons, Journal of the Japan Diabetes Society, 1998, Vol.41, No.8, pp.655-661;
Reference 5: Nobuo, SAKAMOTO and 6 persons, The Japanese Journal of Clinical and Experimental Medicine, 1990, Vol.61, No.1, pp.219-233;
Reference 6: Keiko, FUNAMA and 8 persons, Japanese Pharmacology and Therapeutics, 1997, Vol.25, No.8 pp.2177-2186;
Reference 7: Jean-Louis Chiasson and 5 persons, Lancet, 2002.6, Vol.359, No.9323, pp.2072-2077;
Reference 8: Hiroyuki, ODAKA and 3 persons, Journal of Japanese Society of Nutrition and Food Science, 1992, Vol.45, p.27;
Ref erence 9: Tadao, BABA and 1 person, Supplementary volume of Nippon Rinsho, Ryoikibetsu Shokogun, 1998, No.19, pp.552-554;
Reference 10: Michihiro, KASAHARA and 2 persons, Saishin Igaku, 1996.1, Vol.51, No.1, pp.84-90;
Reference 11: Tomofusa, TSUCHIYA and 1 person, Nippon Rinsho, 1997.8, Vol.55, No.8, pp.2131-2139;
Reference 12: Yoshikatsu, KANAI, Kidney and Dialysis, 1998.12, Vol.45, extra edition, pp.232-237;
Reference 13: E. Turk and 4 persons, Nature, 1991.3, Vol.350, pp.354-356;
Reference 14: Y. Fujita and 5 persons, Diabetologia, 1998, Vol.41, pp.1459-1466;
Reference 15: J. Dyer and 5 persons, Biochemical Society Transactions, 1997, Vol.25, p.479S;
Reference 16: J. Dyer and 4 persons, American Journal of Physiology, 2002.2, Vol.282, No.2, pp.G241-G248;
Reference 17: O. Blondel and 2 persons, Metabolism, 1990, Vol.39, pp.787-793;
Reference 18: A. Khan and 1 person, American Journal of Physiology, 1995, Vol.269, pp.E623-E626;
Reference 19: A. Krook and 6 persons, Diabetes, 1997, Vol.46, pp.2110-2114;
Reference 20: L. Rossetti and 2 persons, Diabetes Care, 1990, Vol.13, pp.610-630;
Reference 21: P. Malathi and 1 person, Biochimica et Biophysica Acta, 1969, Vol.173, pp.245-256;
Reference 22: K. Tsujihara and 6 persons, Chem. Pharm. Bull. (Tokyo), 1996, Vol.44, pp.1174-1180;
Reference 23: M. Watford, Nutrition Reviews, 2002.8, Vol.60, pp.253-264;
Reference 24: M. Shiota and 6 persons, Diabetes, 2002, Vol.51, pp.469-478;
Reference 25: M. Shiota and 4 persons, Diabetes, 1998, Vol.47, pp.867-873;
Reference 26: M. C. Moor and 3 persons, Diabetes Care, 2001, Vol.24, pp.1882-1887;
Reference 27: M. Hawkins and 5 persons, Diabetes, 2002, Vol.51, pp.606-614;
Reference 28: B. W. Wolf and 5 persons, Journal of Nutrition, 2002, Vol.132, pp.1219-1223;
Reference 29: K. F. Petersen and 4 persons, Diabetes, 2001, Vol.50, pp.1263-1268;
Reference 30: P. Malathi and 1 person, Biochimica et Biophysica Acta, 1969, Vol.173, pp.245-256;
Reference 31: K. Tsujihara and 6 persons, Chem. Pharm. Bull. (Tokyo), 1996, Vol.44, pp.1174-1180;
Reference 32: C. P. Corpe and 5 persons, Pflugers Archiv: European Journal of Physiology, 1996, Vol.432, pp.192-201;
Reference 33: Kuniaki, TAKATA, Bio Clinica, 1999, Vol.14, No.10, pp.893-898

### Disclosure of the Invention

The present inventors studied earnestly to find a novel agent exerting an effect caused by fructose ingestion and having a wider range of inhibitory effect on carbohydrate absorption. As a result, it was surprisingly found that a selective SGLT1 inhibitor exerts an excellent hypoglycemic effect and is suitable for the prevention or treatment of a disease associated with hyperglycemia, and thereby, the present invention has been completed.

To be concrete, the present invention relates to
1) an agent for the prevention or treatment of a disease associated with hyperglycemia, comprising as an active ingredient a selective SGLT1 inhibitor;
2) an agent for the prevention or treatment of a disease associated with hyperglycemia, comprising as an active ingredient an SGLT1 inhibitor substantially showing no GLUT2 and/or GLUT5 inhibitory effect;
3) an agent for the prevention or treatment of 1) or 2) described above, wherein the dosage form is an oral agent;
4) an agent for the prevention or treatment of any one of 1) to 3) described above, wherein the disease associated with hyperglycemia is diabetes;
5) an agent for the prevention or treatment of 4) described above, wherein the diabetes is postprandial hyperglycemia;
6) an agent for the prevention or treatment of any one of 1) to 3) described above, wherein the disease associated with hyperglycemia is impaired glucose tolerance (IGT);
7) an agent for the prevention or treatment of any one of 1) to 3) described above, wherein the disease associated with hyperglycemia is diabetic complications;
8) an agent for the prevention or treatment of any one of 1) to 3) described above, wherein the disease associated with hyperglycemia is obesity;
9) a method for the prevention or treatment of a disease associated with hyperglycemia, which comprises administering an effective amount of a selective SGLT1 inhibitor;
10) a method for the prevention or treatment of 9) described above, wherein the selective SGLT1 inhibitor is an SGLT1 inhibitor substantially showing no GLUT2 and/or GLUT5 inhibitory effect;
11) a method for the prevention or treatment of 9) or 10) described above, wherein the dosage form is an oral agent;
12) a method for the prevention or treatment of any one of 9) to 11) described above, wherein the disease associated with hyperglycemia is diabetes;
13) a method for the prevention or treatment of 12) described above, wherein the diabetes is postprandial hyperglycemia;
14) a method for the prevention or treatment of any one of 9) to 11) described above, wherein the disease associated with hyperglycemia is impaired glucose tolerance (IGT);
15) a method for the prevention or treatment of any one of 9) to 11) described above, wherein the disease associated with hyperglycemia is diabetic complications;
16) a method for the prevention or treatment of any one of 9) to 11) described above, wherein the disease associated with hyperglycemia is obesity;
17) a use of a selective SGLT1 inhibitor for the manufacture of a pharmaceutical composition for the prevention or treatment of a disease associated with hyperglycemia;
18) a use of 17) described above, wherein the selective SGLT1 inhibitor is an SGLT1 inhibitor substantially showing no GLUT2 and/or GLUT5 inhibitory effect;
19) a use of 17) or 18) described above, wherein the composition is an oral agent;
20) a use of any one of 17) to 19) described above, wherein the disease associated with hyperglycemia is diabetes;
21) a use of 20) described above, wherein the diabetes is postprandial hyperglycemia;
22) a use of any one of 17) to 19) described above, wherein the disease associated with hyperglycemia is impaired glucose tolerance (IGT);
23) a use of any one of 17) to 19) described above, wherein the disease associated with hyperglycemia is diabetic complications;
24) a use of any one of 17) to 19) described above, wherein the disease associated with hyperglycemia is obesity; and the like.

In the present invention, "selective SGLT1 inhibitor" means a medicament that the active ingredient and/or its metabolite exhibit an SGLT1 inhibitory effect substantially showing no inhibitory effect on absorbing fructose through the small intestine. As the inhibitory effect on absorbing fructose, GLUT2 inhibitory effect, GLUT5 inhibitory effect and the like can be exemplified. As the selective SGLT1 inhibitor, the compounds described in Examples 1 and 2, pharmaceutically acceptable salts thereof and hydrates thereof can be concretely exemplified. Furthermore, the selective SGLT1 inhibitor of the present invention includes other compounds having the above-mentioned effect. Estimation of SGLT1 inhibitory effects in human and the other mammals can be performed an assay method described in the following Example 3 or analogous method thereto. Similarly, estimation of GLUT2 and GLUT5 inhibitory effects can be performed assay methods described in the following References 33 and 34, or analogous method thereto.

In the present invention, as the disease associated with hyperglycemia, diabetes (especially postprandial hyperglycemia), impaired glucose tolerance (IGT), impaired fasting glycemia (IFG), diabetic complications (e.g., retinopathy, neuropathy, nephropathy, ulcer, macroangiopathy), obesity, hyperinsulinemia, hyperlipidemia, hyper-cholesterolemia, hypertriglyceridemia, lipid metabolism disorder, atherosclerosis, hypertension, congestive heart failure, edema, hyperuricemia, gout or the like can be exemplified.

Firstly, the present inhibitors have confirmed the amount of residual fructose in the gastrointestinal tract by performing fructose tolerance test using phlorizin as a known SGLT inhibitor and the compound described in Example 2 of the present invention as an SGLT1 inhibitor. As a result, it was found that the compound of the present invention substantially show no inhibitory effect on absorbing fructose, while phlorizin significantly inhibits absorption of fructose.

Next, in order to investigate whether effects caused by fructose ingestion are present, the present inventors have performed the following test using Zucker fatty fa/fa rats, model animals of type-2 diabetes. As the test drugs, acarbose and miglitol as α-glucosidase inhibitors, phlorizin as an SGLT inhibitor, and the compounds described in Examples 1 and 2 of the present invention as SGLT1 inhibitors were used. In the fructose-containing diet group, mixed carbohydrate (starch: sucrose: lactose = 6:3:1), which corresponds approximately to carbohydrates in regular diets, was loaded. (e.g., see the following Reference 35). On the other hand, in the fructose-free diet group, mixed carbohydrate substituted the corresponding amount of starch for the amount of glucose contained within sucrose, was loaded, because sucrose is a disaccharide and is digested to glucose and fructose in the gastrointestinal tract. Then, comparison study of each diet was performed. As a result, after the sucrose-containing diet administration compared with the sucrose-free diet administration, while α-glucosidase inhibitors and phlorizin did not reduce plasma glucose concentration, compounds of the present invention significantly reduced plasma glucose concentration. From these findings, it was found that a selective SGLT1 inhibitor substantially showing no GLUT2 andGLUT5 inhibitoryeffect significantlyreduces plasma glucose concentration based on fructose ingestion. On the other hand, since α-glucosidase inhibitors reduced fructose absorption by inhibiting of sucrose digestion, and phloretin, which is produced by disesting phlorizin at the gastrointestinal tract, inhibited fructose absorption via its GLUT inhibition, they did not improve plasma glucose concentration at all.

Basing upon the above findings, pharmaceutical compositions comprising as an active ingredient a selective SGLT1 inhibitor have the above-mentioned effect caused by fructose ingestion at regular diets in addition to a wider range of inhibitory effect on carbohydrate absorption. Therefore, they can exhibit a marked hypoglycemic effect. Accordingly, the pharmaceutical compositions of the present invention are extremely suitable as agents for the prevention or treatment of the above-mentioned various diseases associated with hyperglycemia.

Furthermore, in the pharmaceutical compositions of the present invention, a hypoglycemic medicament substantially showing no inhibitory effect on absorbing fructose and/or a medicament for the treatment of diabetic complications other than the selective SGLT1 inhibitor can be suitably comprised or used simultaneously or at different dosage intervals in combination thereto. As the hypoglycemic medicament which can be comprised or used in combination with the compound of the present invention, an insulin sensitivity enhancer (e.g., pioglitazone hydrochloride, rosiglitazone maleate), an SGLT2 inhibitor, a biguanide (e.g., metformin hydrochloride , buformin hydrochloride), an insulin secretion enhancer (e.g., tolbutamide, acetohexamide, tolazamide, glyclopyramide, glybuzole, glyburide/glibenclamide, gliclazide, nateglinide, repaglinide, mitiglinide, glimepiride), an insulin and the like can be exemplified. In addition, as the medicament for the treatment of diabetic complications, an aldose reductase inhibitor (e.g., epalrestat), a sodium channel antagonist (mexiletine hydrochloride), an angiotensin-converting enzyme inhibitor (e.g., imidapril hydrochloride, lisinopril), an angiotensin II receptor antagonist (e.g., potassium losartan, irbesartan), antidiarrhoics or cathartics (e.g., polycarbophil calcium, albumin tannate, bismuth subnitrate) and the like can be exemplified.

As the pharmaceutical compositions employed in the present invention, various dosage forms can be exemplified. Among them, oral pharmaceutical compositions such as tablets, powders, granules, fine granules, capsules, dry sirups, solutions and the like are preferable. The pharmaceutical compositions of the present invention also include sustained release formulations including gastrointestinal mucoadhesive formulations and gastric retention formulations (e.g., see the following References 36 to 39).

These pharmaceutical compositions can be prepared by admixing with or by diluting and dissolving with an appropriate pharmaceutical additive such as excipients, disintegrators, binders, lubricants, diluents, buffers, isotonicities, antiseptics, moistening agents, emulsifiers, dispersing agents, stabilizing agents, dissolving aids and the like, and formulating the mixture in accordance with conventional methods. In case of the uses in combination with the other medicament, they can be prepared by formulating each active ingredient together or individually by analogous methods to that described above.

The dosage of the selective SGLT1 inhibitor in pharmaceutical compositions of the present invention is appropriately decided depending on the sex, age, body weight and degree of symptoms and treatment of each patient. For example, dosages of the compounds described in Examples 1 and 2 are approximately within the range of from 0.1 to 1,000mg per day per adult human in the case of oral administration, and the daily dose can be divided into one to several doses per day and administered suitably. Also, in case uses in combination with the other medicament, the dosage of the compound of the present invention can be decreased, depending on the dosage of the other medicament.
Reference 33: Christopher P. Corpe and 5 persons, Pflugers Arch.-Eur. J. Physiol., 1996, Vol.432, pp.192-201;
Reference 34: Mueckler M and 5 persons, J. Biol. Chem., 1994, Vol.269, No.27, pp.17765-17767;
Reference 35: Yasutoshi, MUTO, "Digestion and Absorption, Adjustment and Adaptation of Gastrointestinal Functions" Daiichishuppan Co., Inc., Tokyo, 1988, pp.228;
Reference 36: International publication No. WO99/10010;
Reference 37: International publication No. WO99/26606;
Reference 38: International publication No. WO98/55107;
Reference 39: International publication No. WO01/97783

### Detailed Description of Drawings

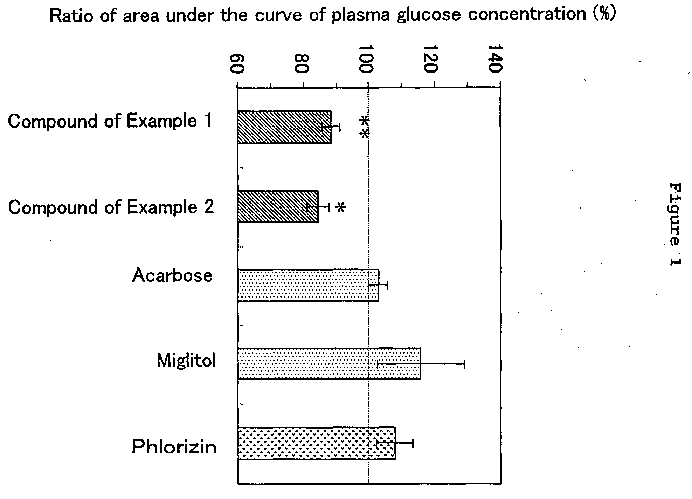

The Figure 1 is a graph showing hypoglycemic effects of each drug caused by fructose ingestion. The vertical axis shows the ratio of area under the curve of plasma glucose comparing sucrose-containing diet with sucrose-free diet (%). The horizontal axis shows each compound, starting from the left, the compound of Example 1, the compound of Example2, acarbose, miglitol and phlorizin, respectively. In the figure, * and ** show significantly difference at P<0.05 and P<0.01, respectively.

### Best Mode for Carrying Out the Invention

The present invention is further illustrated in more detail by way of the following Examples and Test Examples. However, the present invention is not limited thereto.

### Reference Example 1

### 3,5-Dimethoxy-2-(4-nitrobenzoyl)toluene

To a solution of 3,5-dimethoxytoluene (8 g) and 4-nitrobenzoyl chloride (10.7 g) in dichloromethane (150 mL) was added aluminum chloride (7.36 g) under ice-cooling, and the mixture was stirred at room temperature for 14 hours. To the reaction mixture was added ice water. The resulting mixture was poured into 1 mol/L hydrochloric acid, and the organic layer was separated. The organic layer was washed with 1 mol/L hydrochloric acid, 1 mol/L aqueous sodium hydroxide solution and brine successively, and dried over anhydrous magnesium sulfate. The solvent was removed under reduced pressure, and the residue was treated with n-hexane. The precipitated crystals were collected by filtration and dried under reduced pressure to give the title compound (8.72 g).
¹H-NMR (CDCl₃) δ ppm:
2.19 (3H, s), 3.6 (3H, s), 3.86 (3H, s), 6.36 (1H, d, J=1.9Hz), 6.43 (1H, d, J=1.9Hz), 7.92 (2H, d, J=9.2Hz), 8.26 (2H, d, J=9.2Hz)

### Reference Example 2

### 5-Hydroxy-3-methyl-2-(4-nitrobenzoyl)phenol

To a solution of 3,5-dimethoxy-2-(4-nitrobenzoyl)toluene (8.65 g) in dichloromethane (140 mL) was added boron tribromide (6.79 mL) under ice-cooling, and the mixture was allowed to warm to 40°C and stirred for 15 hours. To the reaction mixture was added ice water, and the organic layer was separated. The organic layer was washed with 1 mol/L hydrochloric acid, a saturated aqueous sodium hydrogen carbonate solution and brine successively, and dried over anhydrous magnesium sulfate. The solvent was removed under reduced pressure, and the residue was purified by column chromatography on silica gel (eluent: *n*-hexane/ethyl acetate = 7/1 - 3/1) to give the title compound (6.3 g).
¹H-NMR (CDCl₃) δ ppm:
1.85 (3H, s), 5.83 (1H, s), 6.2-6.3 (1H, m), 6.36 (1H, d, J=2.6Hz), 7.7-7.8 (2H, m), 8.25-8.4 (2H, m), 10.98 (1H, s)

### Reference Example 3

### 5-Methoxycarbonyloxy-3-methyl-2-(4-nitrobenzyl)phenol

To a solution of 5-hydroxy-3-methyl-2-(4-nitrobenzoyl)phenol (1.65 g) and triethylamine (2.1 mL) in tetrahydrofuran (20 mL) was added methyl chloroformate (1.03 mL) under ice-cooling, and the mixture was stirred at room temperature for 4 hours. To the reaction mixture was addedwater, and the resulting mixture was extracted with diethyl ether. The extract was washed with brine, and dried over anhydrous magnesium sulfate. The solvent was removed under reduced pressure to give 3,5-dimethoxycarbonyloxy-2-(4-nitrobenzoyl)toluene (2.37 g). This material was suspended in tetrahydrofuran (20 mL) - water (20 mL). To the suspension was added sodium borohydride (921 mg) under ice-cooling, and the mixture was stirred at room temperature for 2 hours. To the reaction mixture was added a saturated aqueous ammonium chloride solution, and the resulting mixture was extracted with diethyl ether. The extract was washed with a saturated aqueous sodium hydrogen carbonate solution and brine successively, and dried over anhydrous magnesium sulfate. The solvent was removed under reduced pressure, and the residue was purified by column chromatography on silica gel (eluent: *n*-hexane/ethyl acetate = 3/1) to give the title compound (1.62 g).
¹H-NMR (CDCl₃) δ ppm:
2.23 (3H, s), 3.91 (3H, s), 4.1 (2H, s), 5.1-5.25 (1H, brs), 6.5-6.6 (1H, m), 6.6-6.7 (1H, m), 7.3 (2H, d, J=9.1Hz), 8.1 (2H, d, J=9.1Hz)

### Reference Example 4

### 5-Methoxycarbonyloxy-3-methyl-2-(4-nitrobenzyl)phenyl 2,3,4,6-tetra-O-acetyl-β-D-glucopyranoside

To a solution of 5-methoxycarbonyloxy-3-methyl-2-(4-nitrobenzyl)phenol (1 g) and 2,3,4,6-tetra-*O*-acetyl-1-*O*-trichloroacetoimidoyl-α-D-glucopyranose (2.02 g) in dichloromethane (30 mL) was added boron trifluoride-diethyl ether complex (0.2 mL) under ice-cooling, and the mixture was stirred at room temperature for 4 hours. The reaction mixture was poured into water, and the resulting mixture was extracted with ethyl acetate. The extract was dried over anhydrous magnesium sulfate, and the solvent was removed under reduced pressure. The residue was purified by column chromatography on silica gel (eluent: *n*-hexane/ethyl acetate = 2/1 - 3/2) to give the title compound (1.93 g).
¹H-NMR (CDCl₃) δ ppm:
1.75 (3H, s), 2.0 (3H, s), 2.04 (3H, s), 2.08 (3H, s), 2.19 (3H, s), 3.8-4.05 (5H, m), 4.05-4.2 (2H, m), 4.24 (1H, dd, J=12.5Hz, 6.1Hz), 5.05-5.2 (2H, m), 5.2-5.35 (2H, m), 6.75-6.9 (2H, m), 7.21 (2H, d, J=8.6Hz), 8.1 (2H, d, J=8.6Hz)

### Reference Example 5

### 2-(4-Aminobenzyl)-5-methoxycarbonyloxy-3-methylphenyl 2,3,4,6-tetra-O-acetyl-β-D-glucopyranoside

To a solution of 5-methoxycarbonyloxy-3-methyl-2-(4-nitrobenzyl)phenyl 2,3,4,6-tetra-*O*-acetyl-β-D-glucopyranoside (0.61 g) in ethyl acetate (7 mL) was added 10% palladium-carbon powder (0.2 g), and the mixture was stirred at room temperature under a hydrogen atmosphere for 13 hours. The insoluble material was removed by filtration, and the solvent of the filtrate was removed under reduced pressure to give the title compound (0.58 g).
¹H-NMR (CDCl₃) δ ppm:
1.67 (3H, s), 1.99 (3H, s), 2.04 (3H, s), 2.09 (3H, s), 2.17 (3H, s), 3.5 (2H, brs), 3.73 (1H, d, J=15.4Hz), 3.8-3.95 (4H, m), 3.97 (1H, d, J=15.4Hz), 4.1-4.2 (1H, m), 4.24 (1H, dd, J=11.9Hz, 6.0Hz), 5.0-5.2 (2H, m), 5.2-5.35 (2H, m), 6.5-6.6 (2H, m), 6.75-6.85 (4H, m)

### Example 1

### 5-Hydroxy-3-methyl-2-{4-[3-(3-pyridylmethyl)ureido]benzyl}-phenyl β-D-glucopyranoside

To a solution of 2-(4-aminobenzyl)-5-methoxycarbonyl-oxy-3-methylphenyl 2,3,4,6-tetra-*O*-acetyl-β-D-glucopyranoside (0.25 g) and pyridine (0.043 mL) in dichloromethane (10 mL) was added 4-nitrophenyl chloroformate (90 mg), and the mixture was stirred at room temperature for 12 hours. To the reaction mixture were added 3-aminomethylpyridine (0.045 mL) and triethylamine (0.11 mL) , and the mixture was stirred at room temperature for 5 hours. The reaction mixture was concentrated under reduced pressure, and the residue was dissolved in methanol (8 mL). To the solution was added sodium methoxide (28% methanol solution, 0.39 mL), and the mixture was stirred at room temperature for 2 hours. The reaction mixture was concentrated under reduced pressure, and the residue was purified by solid phase extraction on ODS (washing solvent: distilled water, eluent: methanol) and VARIAN BOND ELUT®-SCX (eluent: methanol) successively to give the title compound (0.17 g).
¹H-NMR (CD₃OD) & ppm:
2.11 (3H, s), 3.3-3.5 (4H, m), 3.65-3.75 (1H, m), 3.8-3.95 (2H, m), 4.07 (1H, d, J=15.4Hz), 4.41 (2H, s), 4.8-4.9 (1H, m), 6.32 (1H, d, J=2.2Hz), 6.56 (1H, d, J=2.2Hz), 7.04 (2H, d, J=8.7Hz), 7.17 (2H, d, J=8.7Hz), 7.4 (1H, dd, J=7.8Hz, 5.1Hz), 7.75-7.85 (1H, m), 8.35-8.45 (1H, m), 8.45-8.55 (1H, m)

### Reference Example 6

### [4-(2-Benzyloxyethoxy)-2-methylphenyl]methanol

To a solution of 4-bromo-3-methylphenol (3 g) in *N,N*-dimethylformamide (16 mL) were added cesium carbonate (5.75 g), benzyl 2-bromoethyl ether (2.66 mL) and a catalytic amount of sodium iodide, and the mixture was stirred at room temperature for 16 hours. The reaction mixture was poured into water, and the resulting mixture was extracted with diethyl ether. The organic layer was washed with water, and dried over anhydrous magnesium sulfate. The solvent was removed under reduced pressure to give 4-(2-benzyloxyethoxy)-1-bromo-2-methylbenzene. This material was dissolved in tetrahydrofuran (80 mL). To the solution was added *n*-butyl lithium (2.66 mol/L *n*-hexane solution, 6.63 mL) at -78°C under an argon atmosphere, and the mixture was stirred for 5 minutes. To the reaction mixture was added *N,N*-dimethylformamide (3.09 mL), and the mixture was allowed to warm to 0°C and stirred for 1 hour. The reaction mixture was poured into water, and the resulting mixture was extracted with diethyl ether. The organic layer was washed with water and brine successively, and dried over anhydrous magnesium sulfate. The solvent was removed under reduced pressure to give 4-(2-benzyloxyethoxy)-2-methylbenzaldehyde. This material was dissolved in ethanol (40 mL). To the solution was added sodium borohydride (607 mg) , and the mixture was stirred at room temperature for 3 hours. To the reaction mixture was added methanol, and the resulting mixture was concentrated under reduced pressure. Water was added to the residue, and the mixture was extracted with diethyl ether. The organic layer was washed with a saturated aqueous sodium hydrogen carbonate solution, and dried over anhydrous magnesium sulfate. The solvent was removed under reduced pressure, and the residue was purified by column chromatography on silica gel (eluent: *n*-hexane/ethyl acetate = 6/1 - 1.5/1) to give the title compound (3.34 g).
¹H-NMR (CDCl₃) δ ppm:
1.39 (1H, t, J=5.8Hz), 2.35 (3H, s), 3.8-3.85 (2H, m), 4.1-4.2 (2H, m), 4.6-4.65 (4H, m), 6.73 (1H, dd, J=8.2Hz, 2.6Hz), 6.78 (1H, d, J=2.6Hz), 7.22 (1H, d, J=8.2Hz), 7.25-7.4 (5H, m)

### Reference Example 7

### 4-{[4-(2-Benzyloxyethoxy)-2-methylphenyl]methyl}-1,2-dihydro-5-isopropyl-3H-pyrazol-3-one

To a solution of [4-(2-benzyloxyethoxy)-2-methylphenyl]methanol (3.34 g) in tetrahydrofuran (22 mL) were added triethylamine (1.97 mL) and methanesulfonyl chloride (1. 04 mL) under ice-cooling, and the mixture was stirred for 1 hour. The insoluble material was removed by filtration. The obtained solution of [4-(2-benzyloxyethoxy)-2-methylphenyl]methyl mesylate in tetrahydrofuran was added to a suspension of sodium hydride (60%, 564 mg) and ethyl 4-methyl-3-oxopentanoate (2.13 g) in tetrahydrofuran (40 mL), and the mixture was heated for reflux for 8 hours. To the reaction mixture was added 1 mol/L hydrochloric acid, and the resulting mixture was extracted with diethyl ether. The organic layer was washed with water, and dried over anhydrous magnesium sulfate. The solvent was removed under reduced pressure. To a solution of the residue in toluene (5 mL) was added hydrazine monohydrate (1.79 mL), and the mixture was stirred at 100°C overnight. The reaction mixture was purified by column chromatography on silica gel (eluent: dichloromethane/methanol = 40/1 - 15/1) to give the title compound (3.72 g).
¹H-NMR (CDCl₃) δ ppm:
1.1 (6H, d, J=6.9Hz), 2.3 (3H, s), 2.75-2.9 (1H, m), 3.6 (2H, s), 3.75-3.85 (2H, m), 4.05-4.15 (2H, m), 4.62 (2H, s), 6.64 (1H, dd, J=8.5Hz, 2.5Hz), 6.74 (1H, d, J=2.5Hz), 6.94 (1H, d, J=8.5Hz), 7.25-7.4 (5H, m)

### Reference Example 8

### 3-(2,3,4,6-Tetra-O-acetyl-β-D-glucopyranosyloxy)-4-{[4-(2-benzyloxyethoxy)-2-methylphenyl]methyl}-5-isopropyl-1H-pyrazole

To a solution of 4-{[4-(2-benzyloxyethoxy)-2-methylphenyl]methyl}-1,2-dihydro-5-isopropyl-3*H*-pyrazol-3-one (3.72 g), acetobromo-α-D-glucose (6.03 g) and benzyltri-(*n*-butyl) ammonium chloride (1.52 g) in dichloromethane (18 mL) was added 5 mol/L aqueous sodium hydroxide solution (5.9 mL), and the mixture was stirred at room temperature for 5 hours. The reaction mixture was purified by column chromatography on aminopropylated silica gel (eluent: *n*-hexane/ethyl acetate = 1/1-1/3). The purified material was further purified by column chromatography on silica gel (eluent: *n*-hexane/ethyl acetate = 1/2 - 1/3) to give the title compound (4.33 g).
¹H-NMR (CDCl₃) δ ppm:
1.05-1.15 (6H, m), 1.81 (3H, s), 1.99 (3H, s), 2.02 (3H, s), 2.06 (3H, s), 2.25 (3H, s), 2.7-2.85(1H,m), 3.5 (1H, d, J=16.6Hz), 3.59 (1H, d, J=16.6Hz), 3.75-3.9 (3H, m), 4.05-4.2 (3H, m), 4.3 (1H, dd, J=12.2Hz, 4.1Hz), 4.62 (2H, s), 5.1-5.3 (3H, m), 5.55 (1H, d, J=8.0Hz), 6.6 (1H, dd, J=8.5Hz, 2.5Hz), 6.71 (1H, d, J=2.5Hz), 6.8 (1H, d, J=8.5Hz), 7.25-7.4 (5H, m)

### Reference Example 9

### 3-(2,3,4,6-Tetra-O-acetyl-β-D-glucopyranosyloxy)-4-{[4-(2-hydroxyethoxy)-2-methylphenyl]methyl}-5-isopropyl-1H-pyrazole

3-(2,3,4,6-Tetra-*O*-acetyl-β-D-glucopyranosyloxy)-4-{[4-(2-benzyloxyethoxy)-2-methylphenyl]methyl}-5-isopropyl-1*H*-pyrazole (4.33 g) was dissolved in methanol (24 mL). To the solution was added 10% palladium-carbon powder (800 mg), and the mixture was stirred at room temperature under a hydrogen atmosphere for 8 hours. The insoluble material was removed by filtration, and the solvent of the filtrate was removed under reduced pressure to give the title compound (3.7 g).
¹H-NMR (CDCl₃) δ ppm:
1.1-1.2 (6H, m), 1.83 (3H, s), 1.99 (3H, s), 2.02 (3H, s), 2.06 (3H, s), 2.26 (3H, s), 2.75-2.9 (1H, m), 3.51 (1H, d, J=16.9Hz), 3.59 (1H, d, J=16.9Hz), 3.8-3.85 (1H, m), 3.9-3.95 (2H, m), 4.0-4.1 (2H, m), 4.11 (1H, dd, J=12.5Hz, 2.5Hz), 4.28 (1H, dd, J=12.5Hz, 4.1Hz), 5.1-5.3 (3H, m), 5.55 (1H, d, J=7.9Hz), 6.6 (1H, dd, J=8.3Hz, 2.7Hz), 6.71 (1H, d, J=2.7Hz), 6.82 (1H, d, J=8.3Hz)

### Reference Example 10

### 3-(2,3,4,6-Tetra-O-acetyl-β-D-glucopyranosyloxy)-4-{[4-(2-azidoethoxy)-2-methylphenyl]methyl}-5-isopropyl-1H-pyrazole

To a solution of 3-(2,3,4,6-tetra-*O*-acetyl-β-D-gluco-pyranosyloxy)-4-{[4-(2-hydroxyethoxy)-2-methylphenyl]-methyl}-5-isopropyl-1*H*-pyrazole (1 g) in dichloromethane (10 mL) were added triethylamine (0.34 mL) and methanesulfonyl chloride (0.15 mL), and the mixture was stirred at room temperature for 1 hour. The reaction mixture was poured into 0.5 mol/L hydrochloric acid, and the resulting mixture was extracted with ethyl acetate. The organic layer was washed with water, and dried over anhydrous magnesium sulfate. The solvent was removed under reduced pressure to give 3-(2,3,4,6-tetra-*O*-acetyl-β-D-glucopyranosyloxy)-5-isopropyl-4-({4-[2-(methanesulfonyloxy)ethoxy]-2-methylphenyl}methyl)-1*H*-pyrazole. This material was dissolved in *N,N*-dimethylformamide (7 mL). To the solution was added sodium azide (0.31 g), and the mixture was stirred at 100°C for 3 hours. The reaction mixture was poured into water, and the resulting mixture was extracted with ethyl acetate. The organic layer was washed with water three times, and dried over anhydrous magnesium sulfate. The solvent was removed under reduced pressure, and the residue was purified by column chromatography on silica gel (eluent: *n*-hexane/ethyl acetate = 2/3 - 1/2) to give the title compound (0.79 g).
¹H-NMR (CDCl₃) δ ppm:
1.05-1.2 (6H, m), 1. 82 (3H, s), 2.0 (3H, s), 2.02 (3H, s), 2.06 (3H, s), 2.27 (3H, s), 2.75-2.9 (1H, m), 3.45-3.65 (4H, m), 3.8-3.9 (1H, m), 4.05-4.15 (3H, m), 4.29 (1H, dd, J=12.2Hz, 4.2Hz), 5.1-5.3 (3H, m), 5.56 (1H, d, J=7.7Hz), 6.6 (1H, dd, J=8.3Hz, 2.6Hz), 6.71 (1H, d, J=2.6Hz), 6.82 (1H, d, J=8.3Hz)

### Reference Example 11

### 3-(2,3,4,6-Tetra-O-acetyl-β-D-glucopyranosyloxy)-4-{[4-(2-aminoethoxy)-2-methylphenyl]methyl}-5-isopropyl-1H-pyrazole

To a solution of 3-(2,3,4,6-tetra-*O*-acetyl-β-D-gluco-pyranosyloxy)-4-([4-(2-azidoethoxy)-2-methylphenyl]methyl)-5-isopropyl-1*H*-pyrazole (0.79 g) in tetrahydrofuran (8 mL) was added 10% palladium-carbon powder (50 mg), and the mixture was stirred at room temperature under a hydrogen atmosphere for 1 hour. The insoluble material was removed by filtration, and the solvent of the filtrate was removed under reduced pressure to give the title compound (0.75 g).
¹H-NMR (CDCl₃) δ ppm:
1.05-1.15 (6H, m), 1.82 (3H, s), 2.0 (3H, s), 2.02 (3H, s), 2.06 (3H, s), 2.26 (3H, s), 2.75-2.85 (1H, m), 3.0-3.1 (2H, m), 3.5 (1H, d, J=16.3Hz), 3.59 (1H, d, J=16.3Hz), 3.8-3.9 (1H, m), 3.9-4.0 (2H, m), 4. 12 (1H, dd, J=12.4Hz, 2.4Hz), 4.29 (1H, dd, J=12.4Hz, 4.0Hz), 5.15-5.3 (3H, m), 5.55 (1H, d, J=7.9Hz), 6.59 (1H, dd, J=8.5Hz, 2.6Hz), 6.7 (1H, d, J=2.6Hz), 6.81 (1H, d, J=8.5Hz)

### Example 2

### 3-(β-D-Glucopyranosyloxy)-4-{[4-(2-guanidinoethoxy)-2-methylphenyl]methyl}-5-isopropyl-1H-pyrazole

To a solution of 3-(2,3,4,6-tetra-*O*-acetyl-β-D-gluco-pyranosyloxy)-4-{[4-(2-aminoethoxy)-2-methylphenyl]methyl}-5-isopropyl-1*H*-pyrazole (0.6 g) in tetrahydrofuran (5 mL) - *N,N*-dimethylformamide (1 mL) was added N-(benzyloxycarbonyl)-1*H*-pyrazole-1-carboxamidine (1.89 g), and the mixture was stirred at 60 C° for 20 hours. The reaction mixture was concentrated under reduced pressure, and the residue was purified by column chromatography on silica gel (eluent: *n*-hexane/ethyl acetate = 1/1 - ethyl acetate - ethyl acetate/ethanol = 10/1) to give 3-(2,3,4,6-tetra-*O*-acetyl-β-D-glucopyranosyloxy)-4-({4-[2-(*N'*-benzyloxycarbonyl-guanidino)ethoxy]-2-methylphenyl}methyl)-5-isopropyl-1*H*-pyrazole (0.31 g). This material was dissolved in methanol (6 mL). To the solution was added sodium methoxide (28% methanol solution, 0.023 mL), and the mixture was stirred at room temperature for 1 hour. The reaction mixture was concentrated under reduced pressure, and the residue was purified by solid phase extraction on ODS (washing solvent: distilled water, eluent: methanol) to give 4-({4-[2-(*N*'-benzyloxycarbonyl-guanidino)ethoxy]-2-methylphenyl}methyl)-3-(β-D-gluco-pyranosyloxy)-5-isopropyl-1*H*-pyrazole(0.2 g). This material was dissolved in methanol (3 mL). To the solution was added 10% palladium-carbon powder (50 mg), and the mixture was stirred at room temperature under a hydrogen atmosphere for 1 hour. The insoluble material was removed by filtration, and the solvent of the filtrate was removed under reduced pressure to give the title compound (0.15 g).
¹H-NMR (CD₃OD) δ ppm:
1.05-1.15 (6H, m) , 2.3 (3H, s), 2.75-2.9 (1H, m), 3.25-3.4 (4H, m), 3.55 (2H, t, J=5.0Hz), 3.6-3.75 (3H, m), 3.75-3.85 (1H, m), 4.06 (2H, t, J=5.0Hz), 5.02 (1H, d, J=7.0Hz), 6. 65 (1H, dd, J=8.5Hz, 2.6Hz), 6.75 (1H, d, J=2.6Hz), 6.88 (1H, d, J=8.5Hz)

### Example 3

### Assay for inhibitory effects on rat SGLT1 activity

### 1) Cloning and construction of the vector expressing rat SGLT1

Using rat kidney cDNA library (QUICK-Cline™ cDNA ; Clontech) as template, the DNA fragment coding 111 to 2203 bp of rat SGLT1 (ACCESSION : M16101), which was reported by Kasahara et al. , was amplified by PCR method and inserted into the SrfI site of pCMV-Script (Stratagene). The DNA sequence inserted was perfectly matched to the previously reported sequence on the amino acids level.

### 2) Establishment of cell line stably expressing rat SGLT1

The expression vector of rat SGLT1 was digested with MluI into a linear DNA. The linear DNA was transfected into CHO-K1 cells by means of lipofection (Superfect Transfection Reagent: QIAGEN). Neomycin resistant cell lines were selected by culture in the medium containing G418 (1 mg/mL, LIFE TECHNOLOGIES), and then the activity against the uptake of methyl-α-D-glucopyranoside was measured by the method described below. The cell line, which showed the greatest uptake activity, was selected and designated as CrS1. CrS1 cells were cultured in the presence of G418 at 200 µg/mL.

### 3) Measurement of the inhibitory activity against the uptake of methyl-α-D-glucopyranoside (α-MG)

CrS1 cells were seeded into a 96-well culture plate at a density of 3 × 10⁴ cells/well and cultured in the presence of G418 at 200 µg/mL for 2 days, and were used in the uptake assay. A mixture of non-labeled (Sigma) and ¹⁴C-labeled α-MG (Amersham Pharmacia Biotech) was added to the uptake buffer (pH 7. 4; containing 140 mM sodium chloride, 2 mM potassium chloride, 1 mM calcium chloride, 1 mM magnesium chloride, 10 mM 2-[4-(2-hydroxyethyl)-1-piperazinyl]ethane sulfonic acid, and 5 mM tris(hydroxymethyl)aminomethane) at the final concentration of 1 mM. A test compound was dissolved in dimethyl sulfoxide, and then appropriately diluted with distilled water. The test compound solution was added to the uptake buffer containing 1 mM α-MG, and designated as a measurement buffer. For the control group, the measurement buffer without any test compound was prepared. For measuring the basal uptake, a basal uptake measurement buffer, which contains 140 mM chorine chloride instead of sodium chloride, was prepared. After removing the culture medium of CrS1 cells, 180 µL of the pre-treatment buffer (the basal uptake buffer without α-MG) was added to each well and incubated at 37° C for 10 minutes. After repeating the same treatment, the pre-treatment buffer was removed. To each well was added 75 µL of the measurement buffer or the basal uptake buffer was added and incubated at 37° C for 1 hour. After removing the measurement buffer, cells were washed twice with 180 µL per well of the washing buffer (the basal uptake buffer containing 10 mM non-labeled α-MG). The cells were solubilized by 75 µL per well of 0.2 mol/L sodium hydroxide. The cell lysates were transferred into PicoPlates (Packard), and then added 150 µL of MicroScint-40 (Packard) and mixed. Radioactivity was measured by means of micro-scintillation counter TopCount (Packard). One hundred % was set to the difference between the uptake in the control group and the basal uptake, and the uptake of methyl α-D-glucopyranoside at each drug concentration were calculated. The drug concentration, at which 50% uptake of methyl α-D-glucopyranoside was inhibited (IC₅₀ value), was calculated using logit plot. The results are shown in Table 1.

**[Table 1]**

| Test Compound | IC₅₀ value (nM) |
|---|---|
| Example 1 | 139 |
| Example 2 | 38.1 |
| Phlorizin | 191 - 316 |

### Test Example 1

### Effects of an SGLT1 inhibitor on absorption of fructose

The male Wistar rats (aged 8 weeks old) were orally administered a compound of Example 2 (0.3 mg/kg) or phlorizin (40, 100 mg/kg). Immediately after the administration, 0.2 g/kg of fructose was loaded. After 30 minutes, rats were sacrificed with exsanguination under ether anesthesia and then the contents of stomach and small intestine were collected with 10 mL ice-cold saline. Fructose concentration was measured by fructose assay kit (D-glucose / D-Fructose; Roche diagnostics) and calculated residual fructose (% of dose) in the gastrointestinal tract. Statistical analysis between control and treated groups are calculated using T test. The results are shown in Table 2. In the Table, ** and *** are showed significantly difference at P<0.01 and P<0.001, respectively.

**[Table2]**

| Test Compound | Residual fructose (%) |
|---|---|
| Control | 11.8 ± 2.1 |
| Example 2 | 12.2 ± 2.6 |
| Phlorizin (40 mg/kg) | 22.5 ± 1.9 ** |
| Phlorizin (100 mg/kg) | 32.2 ± 1.5 *** |

### Test Example 2

### Effects of SGLT1 inhibitors, an SGLT inhibitor and α-glucosidase inhibitors after mixed-carbohydrate containing sucrose loading.

After overnight fasting, the male Zucker fa/fa rats (aged 15-17 weeks old) were orally administered an SGLT1 inhibitor (the compound of Example 1, 0.5 mg/kg; the compound of Example 2, 0.3 mg/kg), α-glucosidase inhibitor (acarbose: 5 mg/kg, miglitol: 5 mg/kg) or an SGLT inhibitor (phlorizin, 100 mg/kg). Immediately after the compound administration, mixed-carbohydrate with sucrose (starch : sucrose : lactose = 6 : 3 : 1) or without sucrose (starch : lactose = 7.5 : 1) was loaded at a dose of 1.6 g glucose/kg. The blood was collected immediately before and after the administration with the time course (0, 0.5, 1, 2, 3 hours). The plasma was collected to quantify the plasma glucose concentration. The area under the curve of plasma glucose concentration from 0 to 3 hours was calculated by the trapezoidal method. Statistical analysis was T test between existence and nonexistence of sucrose in mixed-carbohydrate solution on each compound. The results are shown in Figure 1.

### Industrial Applicability

The pharmaceutical compositions of the present invention comprising as an active ingredient a selective SGLT1 inhibitor have the above-mentioned effect caused by fructose ingestion at regular diets in addition to a wider range of inhibitory effect on carbohydrate absorption. Therefore, they can exhibit a marked hypoglycemic effect. Accordingly, the pharmaceutical compositions are extremely suitable as agents for the prevention or treatment of the above-mentioned various diseases associated with hyperglycemia.

## Claims

1. An agent for the prevention or treatment of a disease associated with hyperglycemia, comprising as an active ingredient a selective SGLT1 inhibitor.

2. An agent for the prevention or treatment as claimed in claim 1, wherein the active ingredient is an SGLT1 inhibitor substantially showing no GLUT2 and/or GLUT5 inhibitory effect.

3. An agent for the prevention or treatment as claimed in claim 1 or 2, wherein the dosage form is an oral agent.

4. An agent for the prevention or treatment as claimed in any one of claims 1-3, wherein the disease associated with hyperglycemia is diabetes.

5. An agent for the prevention or treatment as claimed in claim 4, wherein the diabetes is postprandial hyperglycemia.

6. An agent for the prevention or treatment as claimed in any one of claims 1 to 3, wherein the disease associated with hyperglycemia is impaired glucose tolerance.

7. An agent for the prevention or treatment as claimed in any one of claims 1 to 3, wherein the disease associated with hyperglycemia is diabetic complications.

8. An agent for the prevention or treatment as claimed in any one of claims 1 to 3, wherein the disease associated with hyperglycemia is obesity.

9. A method for the prevention or treatment of a disease associated with hyperglycemia, which comprises administering an effective amount of a selective SGLT1 inhibitor.

10. A method for the prevention or treatment as claimed in claim 9, wherein the selective SGLT1 inhibitor is an SGLT1 inhibitor substantially showing no GLUT2 and/or GLUT5 inhibitory effect.

11. A method for the prevention or treatment as claimed in claim 9 or 10, wherein the dosage form is an oral agent.

12. A method for the prevention or treatment as claimed in any one of claims 9 to 11, wherein the disease associated with hyperglycemia is diabetes.

13. A method for the prevention or treatment as claimed in claim 12, wherein the diabetes is postprandial hyperglycemia.

14. A method for the prevention or treatment as claimed in any one of claims 9 to 11, wherein the disease associated with hyperglycemia is impaired glucose tolerance.

15. A method for the prevention or treatment as claimed in any one of claims 9 to 11, wherein the disease associated with hyperglycemia is diabetic complications.

16. A method for the prevention or treatment as claimed in any one of claims 9 to 11, wherein the disease associated with hyperglycemia is obesity;

17. A use of a selective SGLT1 inhibitor for the manufacture of a pharmaceutical composition for the prevention or treatment of a disease associated with hyperglycemia.

18. A use as claimed in claim 17, wherein the selective SGLT1 inhibitor is an SGLT1 inhibitor substantially showing no GLUT2 and/or GLUT5 inhibitory effect.

19. A use as claimed in claim 17 or 18, wherein the composition is an oral agent.

20. A use as claimed in any one of claims 17 to 19, wherein the disease associated with hyperglycemia is diabetes.

21. A use as claimed in claim 20, wherein the diabetes is postprandial hyperglycemia.

22. A use as claimed in any one of claims 17 to 19, wherein the disease associated with hyperglycemia is impaired glucose tolerance.

23. A use as claimed in any one of claims 17 to 19, wherein the disease associated with hyperglycemia is diabetic complications.

24. A use as claimed in any one of claims 17 to 19, wherein the disease associated with hyperglycemia is obesity.
